# EUROPEAN PATENT APPLICATION

(11) **EP 4 197 395 A1**
(43) Date of publication of application: **21.06.2023**
(21) Application number: 22213476.9
(22) Date of filing: 14.12.2022
(51) Int. Cl.: A47C 1/00, A47C 7/38, A47C 20/00, A61F 5/37

(54) **HEAD SUPPORT ARRANGEMENT**

(30) Priority: 16.12.2021 NO 20211515
(71) Applicant: Schjetlein, Ole Jakob, 1395 Hvalstad (NO)
(72) Inventor: Schjetlein, Ole Jakob, 1395 Hvalstad (NO)
(74) Representative: Onsagers AS

(57) **Abstract**

The invention describes a support arrangement for the head of people sitting in a sitting furniture with a backrest. The support arrangement comprises an elongated back band (1) placed between the user's back and the backrest to provide friction and an elongated flexible headband (2) placed against the user's upper and front part of the forehead and along respective sides of the head towards the back and which is attached directly or indirectly to an upper edge (9) of the back band (1). The headband (2) has a width at the forehead that covers an area of movement of a movable balance point (4) on the head where a holding force (F) from the back band (1), which is transferred to the head by means of the headband, at all times hits essentially perpendicular on a tangent plane (10) to the head, when the tangent plane contains the balance point (4).

## Description

### Field of the invention

The present invention relates to a support arrangement for the head of a human being. For example, for use when travelling, more specifically to support the forehead of a user sitting in a seat on a train or plane, or whenever there is a need to sit and sleep. It can also be used by people with disabilities who sit in a wheelchair, to support the head if there is a lack of muscle strength in the neck, e.g. in the case of MS.

### Background

Several such devices are known which are designed to support a user who is sitting, e.g. in an airplane seat and who wants to find a comfortable position to sleep in. What is sought to be achieved is to support the user so that the head does not fall forward or to the side so that you wake up, or that you wake up with a pain in the neck.

From patent application US 2004/245832, a device is known in the form of a support arrangement for the head comprising a post which is placed behind the user's back. The pole extends from the lower back and up behind the user's head. At the upper end of the post, a band is attached that goes around the user's head and holds it up when the person sleeps. This device and the design-protected USD 670035S have been copied according to the application and studied, and they turn out to have some significant drawbacks. With the first device, the disadvantage is that the head is locked, with the face straight ahead. Most people will find this uncomfortable when going to sleep. The construction with a rigid bar along the back makes it impossible to adjust the headband and thus the position of the head. Another disadvantage is that the device is difficult to transport.

From design application US D670035 S, a similar device is known, with a band to be positioned behind the user's back. From the drawings, it looks like the band, and the rest of the device is made of leather. To keep the band in place, a plate is provided in a lower part of the band. A mask is attached to the upper part of the band, which goes around the user's forehead and also covers the eyes. This has been done to prevent the headband from sliding up and backwards. The "sleep mask part" cannot be opted out. To prevent the mask from sliding down, there is a strap that stretches across the middle of the user's head. This is not comfortable and ruins the hairstyle. In addition, the back band is so narrow that there is too little lateral support for the head. This headband seems to block out air, because it consists of two layers. The outermost layer is probably made of leather, while the innermost is made of twice as thick fabric, probably in a soft material. This will make the headband hot and sticky.

The fact that the headband is made single curved means that the pressure on the forehead is unevenly distributed on the forehead, which is a double curved surface. It will all take up a relatively large amount of space. Experiments show that the design of the headband in particular is a weakness of previously known support arrangements for heads. The result is that the head can get a locked position where it is difficult to get the necessary freedom to e.g. to read, and that the head is not supported evenly and comfortably.

The principles behind the invention have some similarities with the headbands the Sherpas in Nepal use when carrying goods. The headband is connected to the goods by means of rope or a teat or similar. A sherpa 'balances' the headband on a balance point on the forehead where the forces from the load act essentially perpendicular to a tangent plane to the forehead. Furthermore, the forces are transferred along the rope on both sides of the head. The plane through the two load-bearing ropes will, due to human anatomy, hit close to the joint between the spine and skull so that the spine is loaded directly from above. If the 'balancing' fails, the headband will be pulled off the forehead in a forward or backward direction.

An object of the invention is to produce a support arrangement for the head which provides a comfortable support for the head when the user sits in a sitting furniture with a backrest.

Another object of the invention is to provide a head support arrangement which can be folded into a small compact unit which is easy to carry.

### Summary of the invention

The invention comprises a support arrangement for the head of people sitting in a sitting furniture with a backrest. The support arrangement comprises an elongated back band and a headband. In a working position, the back band is placed between the user's back and the backrest to provide friction. Furthermore, the support arrangement comprises an elongated flexible headband which in a working position is placed against the user's upper and front part of the forehead and is placed along respective sides of the head in the direction of the back, and which is attached directly or indirectly to an upper edge of the back band. In the working position, the headband has a width at the forehead that covers an area of movement of a movable balance point on the head where a holding force from the back band, which is transferred to the head by means of the headband, at all times strikes essentially perpendicular to a tangent plane to the head containing the balance point.

In one embodiment of the support arrangement, the back band is made of a flexible material with relatively high tensile strength. The flexibility of the material allows the back band to be folded up and the high tensile strength allows the frictional forces between the back of the user and the backrest to be transferred to the headband. By relatively high tensile strength we mean that the back band should not stretch much during use. We do not mean that the back band should handle large forces.

In another embodiment of the support arrangement, the headband is made of an elastic material.

In a further embodiment of the support arrangement, the headband has a front and rear outer edge with less elasticity than the rest of the headband.

In a further embodiment of the support arrangement, the reduced elasticity of the front and rear outer edges is achieved by the edges being folded two or more times and sewn onto itself.

In a further embodiment of the support arrangement, the headband has a concave design to follow the shape of the forehead.

In a further embodiment of the support arrangement, the extent of the headband in the working position comprises at least a triangle on each side of the head defined by the respective extreme point of the width of the headband at the balance point and a point of intersection below a point of rotation between the skull and the spine on respective sides of the head, where the point of intersection lies on the midline of the headband.

In a further embodiment of the support arrangement, the headband is attached indirectly to the back band with a fastening cord to either side of an upper edge of the back band.

In a further embodiment of the support arrangement, the fastening cord can be tied in different lengths.

In a further embodiment of the support arrangement, the back band is made of a mesh material with sufficient stiffness so that the mesh material is held flat between the seat back and the user's back.

In a further embodiment of the support arrangement, the back band is equipped with a pocket into which the support arrangement can be folded.

### Brief description of the drawings

In order to improve the understanding of the invention, some drawings have been made where the same reference number refers to the same feature in different figures.
Fig. 1 shows an embodiment of the head support arrangement used by a user sitting in a chair seen from the side.
Fig. 2 shows the same as Fig. 1 seen from behind.
Fig. 3 shows different designs of the headband.
Fig. 4 shows the anatomy of the head and neck interacting with the support arrangement.
Fig. 5a and b show some more details of the support arrangement.

### Detailed description

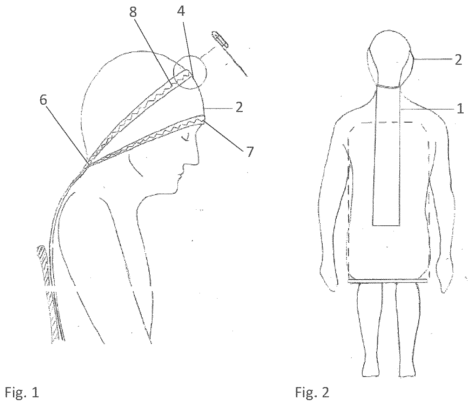

Directions in this description refer to a situation where a user uses the product sitting in a sitting furniture as shown in fig. 1. The invention is a support arrangement for the head for sleeping in a sitting furniture, typically during long journeys where you are sitting in a seat for a long time.

The support arrangement for the head comprises an elongated, preferably flexible back band 1 with relatively high tensile strength which is to be placed between the user's back and a chair seat or a backrest to provide friction as shown in fig. 1 and 2. People have many sizes and shapes, and in order for the back band 1 to be usable by everyone, the length should be at least 40 cm, preferably at least 50 cm. The back band must provide a frictional force and the material used should be able to maintain its shape during use so that the frictional surface and thus the frictional force that can be extracted is not reduced. A relatively stiff mesh material or a textile with reinforcements are good solutions. Furthermore, the back band 1 must be foldable or rollable so that it can be packed into a pocket. The width should be over 10 cm to achieve a sufficient friction surface and a wider back band provides better resistance to lateral movements of the head. On the other hand, a wider back band provides a larger volume of the head support arrangement when packed. We have found that widths between 8 and 15 cm give the desired effect, while the support arrangement for the head still can be packed into a size that is easy to carry in a pocket or hand luggage.

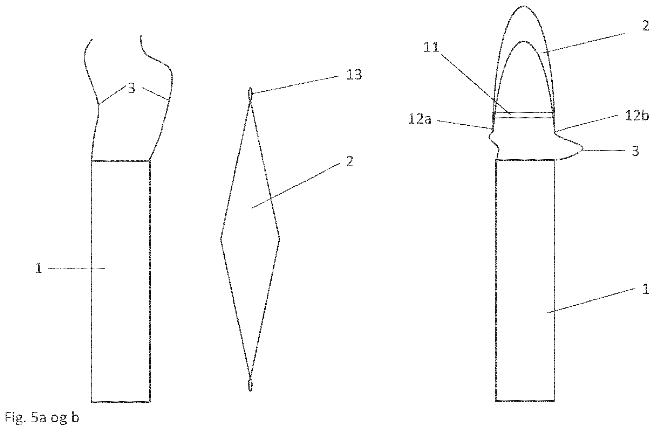

The support arrangement for the head further comprises an elongated flexible headband 2 to be placed against the user's upper and front part of the forehead and is guided, preferably symmetrically, along respective sides of the head towards the back. The headband is attached directly or indirectly to an upper edge 9 of the back band 2. Preferably, the headband 2 is attached to the back band 1 with a fastening cord 3 on each side of the head which can be tied in different lengths to adapt to the user's size. The headband can be equipped with loops 13 on respective ends 12a and b of the headband as shown in fig. 5a for easy connection to the cords 3 that can be attached to the back band 1.

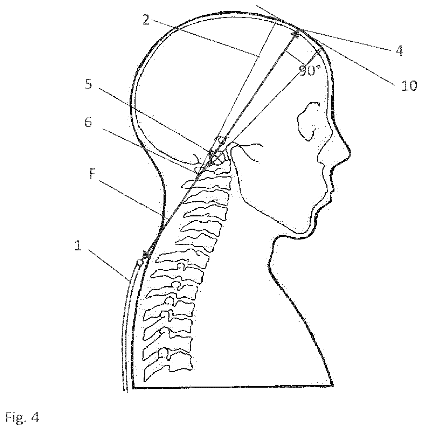

The headband 2 covers an area of motion of a movable balance point 4 on the forehead. The balance point 4, indicated in fig. 1 and fig. 4 is the point on the user's forehead where a holding force F, shown in fig. 4, from the back band hits essentially perpendicular on a tangent plane to the forehead when positioned at the balance point 4. As the user's head tilts forward when the user falls asleep, the balance point will tend to shift somewhat due to changing angles. When the headband 2 covers the area near the balance point 4, there will be sufficient friction in the headband so that the headband does not slip. The user must therefore place the headband 2 on the forehead so that the balance point is covered. In practice, the user will place the headband on the forehead and pull it in the opposite direction if the user feels that it is slipping. After a few adjustments, the headband will fit correctly.

As mentioned, the balance point will move somewhat with movements of the head and it is desirable that the balance point lies within the headband 2. Therefore, it is beneficial to have a width of the headband 2 at the forehead so that an area of movement of the balance point is covered when the head is tilting forward when falling asleep. The balance point 4 is a balance point where the headband is neither pulled backwards nor forwards.

Preferably, the headband is made of an elastic material so that the holding force F from the back band is evenly and comfortably distributed and it is possible to move the head without the user experiencing sudden stops in desired movements.

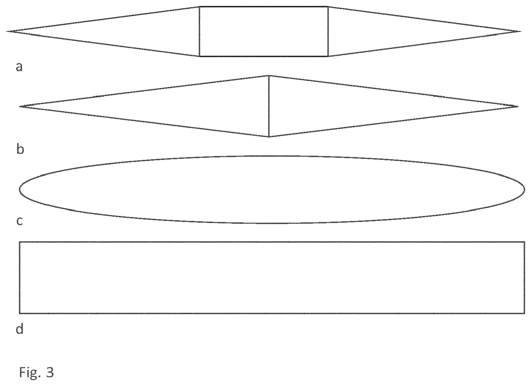

A headband can have many shapes as shown in fig. 3. In an embodiment shown in fig. 4, the headband's extent, in the position of use, includes at least a triangle from the respective outer extreme points of the headband's width at the balance point 4 and a crossing point 6 below a rotational point 5 between the skull and the spine on respective sides of the head where a front outer edge 7 of the headband meets the rear outer edge 8 of the headband. The crossing point 6 is preferably on a center line of the headband 2. The advantage of a relatively low crossing point 6 is that the forces from the front and rear outer edges 7, 8 of the headband is directed more essentially perpendicular to the skull and increase friction. At the same time, the elasticity of the material will give greater freedom to lateral movements of the head at a relatively low crossing point. The combination of elasticity in the headband 2, the holding power of the back band 1 and the human anatomy around the head, neck and back will provide a possibility of movement laterally and forwards which provides an increasing resistance to increasing movements.

In an embodiment indicated in Figure 1, the headband has a front and rear outer edge with less elasticity than the rest of the headband. The reduced elasticity of the front and rear outer edges 7, 8 can be obtained, for example, by means of the edges being folded two or more times and sown on to themselves, as shown in fig. 1.

The length of the headband should at least go from one earlobe, over the forehead, and to the other earlobe. In other words, the headband should at least extend from a horizontal line in the direction of shoulder to shoulder through the rotational point 5 between the skull and spine, over the balance point in the forehead and down to said horizontal line on the other side of the head. It corresponds to approx. 40 cm for an adult. Preferably, the length of the headband is between 30 and 70 cm, more preferably between 45 and 55 cm.

As mentioned, the width of the band must cover the balance point 4 as it moves on the forehead when the head moves. A wider band provides a better distribution of holding forces and a greater likelihood of covering the balance point when the user puts on the head support arrangement. However, the holding forces are not that great and tests show that a good effect is achieved with headbands with widths from 5 to 15 cm. More preferably, the width of the headband at the balance point 4, that is in the middle of the headband, is between 8 and 12 cm.

In one embodiment, the headband has a concave design to follow the shape of the forehead. This helps preventing the headband from slipping out of position. The material in the headband must have friction against the forehead. Slippery materials will perform inferior to materials with high friction.

It is advantageous to tighten/slacken the cords 3 so that the upper edge 9 of the back band is at the neck or close to the neck. If the cords are too long, they tend to slide sideways towards the shoulders as the head tilts forward and much of the holding effect disappears. To prevent the cords from sliding sideways/forwards, it may be advantageous to have a holding part 11 between two ends, 12 a and b, of the headband as shown in fig. 5b. The holding part 11 can be, for example, a cord or a band.

### Reference list

1 Back band
2 Headbands
3 Fastening cord
4 Balance point
5 Rotational point between skull and spine
6 Crossing point
7 Front outer edge of the headband
8 Rear outer edge of the headband
9 Upper edge of the back band
10 Tangent plane to the head
11 Holding part
12 a and b Ends of the headband
13 Loop to attach to the fastening cord 3

## Claims

1. Support arrangement for the head of people sitting in a sitting furniture with a backrest, where the support arrangement comprises:
an elongated back band (1) which, in a working position, is placed between the user's back and the backrest to provide friction,
an elongated flexible headband (2) which, in a working position, is placed against the user's upper and front part of the forehead and is placed along respective sides of the head in a direction towards the back and which is attached directly or indirectly to an upper edge (9) of the back band (1),
wherein the headband (2) in the working position has a width at the forehead that covers an area of movement of a movable balance point (4) on the head where a holding force (F) from the back band (1), which is transferred to the head by means of the headband, hits at all times essentially perpendicular to a tangent plane (10) of the head, when the tangent plane contains the balance point (4).

2. Support arrangement for head according to claim 1, where the back band (1) is made of a flexible material with high tensile strength.

3. Support arrangement for head according to claim 1 or 2, where the headband (2) is made of an elastic material.

4. Support arrangement for head according to claim 3, where the headband (2) has a front and rear outer edge (7, 8) with less elasticity than the rest of the headband.

5. Support arrangement for head according to claim 4, where the reduced elasticity of the front and rear outer edge (7, 8) results from the edges being folded two or more times and sown onto itself.

6. Support arrangement for head according to any one of the preceding claims, where the headband (2) has a concave design to follow the shape of the forehead.

7. Support arrangement for head according to any one of the preceding claims, where the extent of the headband in the position of use includes at least a triangle on each side of the head defined by the respective extreme point of the width of the headband at the balance point (4) and a crossing point (6) below a rotation point (5) between the skull and the spine on respective sides of the head, where the crossing point (6) is on the center line of the headband ((2).

8. Support arrangement for head according to any one of the preceding claims, where the headband (2) is attached indirectly to the back band with a fastening cord (3) to each side of an upper edge (9) of the back band.

9. Support arrangement for head according to any one of the preceding claims where the fastening cord (3) can be tied in different lengths.

10. Support arrangement for head according to any one of the preceding claims, where the back band (1) is made of a mesh material with sufficient stiffness to keep the mesh material flat between the seat back and the user's back.

11. Support arrangement for head according to claim 1, where the back band (1) is equipped with a pocket into which the support arrangement can be folded.
